(19) Europäisches Patentamt
European Patent Office
Office européen des brevets

(11) Veröffentlichungsnummer: **0 156 036**
**A2**

(12) # EUROPÄISCHE PATENTANMELDUNG

(21) Anmeldenummer: 84116414.8

(22) Anmeldetag: 27.12.84

(51) Int. Cl.⁴: **C 08 G 77/20**
C 08 L 83/07, C 08 J 3/28

(30) Priorität: 27.03.84 DE 3411284

(43) Veröffentlichungstag der Anmeldung:
02.10.85 Patentblatt 85/40

(84) Benannte Vertragsstaaten:
BE DE FR GB IT NL

(71) Anmelder: WACKER-CHEMIE GMBH
Prinzregentenstrasse 22
D-8000 München 22(DE)

(72) Erfinder: Preiner, Gerhard, Dr. Dipl. Chem.
Karl-Gross-Strasse 17
D-8263 Burghausen(DE)

(72) Erfinder: Louis, Eckhart, Dr. Dipl. Chem.
Angererweg 20
D-8263 Burghausen(DE)

(72) Erfinder: Müller, Johann
Friedrich-Ebert-Strasse 3
D-8263 Burghausen(DE)

(54) **Organo(poly)siloxane und derartige Organo(poly)siloxane enthaltende, durch Bestrahlung mit Licht vernetzbare Massen.**

(57) Organo(poly)siloxane mit Monoorganosiloxaneinheiten der Formel

$$H_2C=CR^1COO(CH_2)_nSiO_{3/2} ,$$

worin $R^1$ Wasserstoff oder einen Alkylrest mit 1 bis 4 Kohlenstoffatomen und n eine ganze Zahl im Wert von 2, 3 oder 4 bedeutet, die 1 bis 60 Molprozent Einheiten der Formel

$$R_3SiO_{1/2} ,$$

worin R gleiche oder verschiedene SiC-gebundene organische Reste bedeutet, enthalten, wobei die restlichen Einheiten mindestens zu 95 Molprozent Monoorganosiloxaneinheiten der oben angegebenen Formel sind. Derartige Organo(poly)siloxane eignen sich ausgezeichnet als wesentliche Bestandteile von durch Bestrahlung mit Licht vernetzbare Massen.

EP 0 156 036 A2

W A C K E R - C H E M I E
G M B H



München, den 22.2.1984
Dr.Ru/rei

Wa 8333-S

# Organo(poly)siloxane und derartige Organo(poly)-siloxane enthaltende, durch Bestrahlung mit Licht vernetzbare Massen

Aus GB 20 23 628 A (veröffentlicht 3. Januar 1980, Minnesota Mining and Manufacturing Company) sind oligomere methacrylsubstituierte Alkylsiloxane und derartige Organosiliciumverbindungen enthaltende, durch Bestrahlung mit Licht vernetzbare Massen bereits bekannt.

Es bestand nun die Aufgabe, Organo(poly)siloxane bereitzustellen, die besonders leicht zugänglich und besonders lange lagerfähig sind, eine besonders niedrige Viskosität haben und mit denen durch Bestrahlung mit Licht vernetzbare Massen bereitet werden können, in die besonders leicht oder besonders viel Füllstoff mit großer Oberfläche eingearbeitet werden kann und die nach Vernetzung sich nicht unter weiterem Lichteinfluß verfärbende Produkte mit besonders hoher Druckfestigkeit und besonders niedriger Wasseraufnahme und besonders niedrigem Ausdehnungskoeffizient ergeben. Diese Aufgabe wird durch die Erfindung gelöst.

Gegenstand der Erfindung sind Organo(poly)siloxane mit Monoorganosiloxaneinheiten der Formel

$$H_2C{=}CR^1COO(CH_2)_nSiO_{3/2}\quad ,$$

worin $R^1$ Wasserstoff oder einen Alkylrest mit 1 bis 4 Kohlenstoffatomen und n eine ganze Zahl im Wert von 2,3 oder 4 bedeutet, dadurch gekennzeichnet, daß sie 1 bis 60 Molprozent Einheiten der Formel

$$R_3SiO_{1/2}$$ ,

worin R gleiche oder verschiedene SiC-gebundene organische Reste bedeutet, enthalten, wobei die restlichen Einheiten mindestens zu 95 Molprozent Monoorganosiloxaneinheiten der oben angegebenen Formel sind.

Vorzugsweise enthalten die erfindungsgemäßen Organo(poly)siloxane 0,5 bis 5 Gewichtsprozent Si-gebundene Hydroxylgruppen.

Gegebenenfalls können die erfindungsgemäßen Organo(poly)siloxane auch bis zu 2 Gewichtsprozent SiOC-gebundene organische Reste enthalten. Beispiele für SiOC-gebundene organische Reste sind (unter Einbeziehung des Si-gebundenen Sauerstoffs) der Methoxy-, Ethoxy- und Methoxyethylenoxyrest.

Beispiele für Alkylreste $R^1$ sind der Methyl-, Ethyl-, n-Propyl-, Isopropyl-, n-Butyl- und sec.-Butylrest. Vorzugsweise ist $R^1$ Wasserstoff oder der Methylrest. Am meisten ist bevorzugt, daß $R^1$ der Methylrest ist.

Vorzugsweise hat n den Wert 3.

Beispiele für organische Reste R sind Kohlenwasserstoffreste, wie Alkylreste, wobei die oben genannten Beispiele für Alkylreste $R^1$ ausnahmslos auch für Alkylreste R gelten, cycloaliphatische Kohlenwasserstoffreste, wie der Cyclohexylrest; Kohlenwasserstoffreste mit aliphatischen Mehrfachbindungen, wie der Vinyl- und Allylrest; Arylreste, wie der Phenylrest; Alkarylreste, wie Tolylreste; und Aralkylreste, wie der Benzylrest. Die Kohlenwasserstoffreste R können auch Substituenten, welche eine Vernetzung der Organo(poly)siloxane durch Bestrahlung mit Licht nicht beeinträchtigen, aufweisen. Solche Substituenten sind z.B. Chloratome, sodaß ein weiteres Beispiel für R der gamma-Chlorpropylrest ist. Vorzugsweise sind

jedoch schon wegen der leichteren Zugänglichkeit alle R jeweils Methylreste.

Vorzugsweise enthalten die erfindungsgemäßen Organo(poly)siloxane 15 bis 25 Molprozent Einheiten der Formel

$$R_3SiO_{1/2}$$

und 2 bis 5 Gewichtsprozent Si-gebundene Hydroxylgruppen. Sie haben dann eine Viskosität in einem nicht allzu breiten Bereich um 1000 mPa.s bei 25°C.

Vorzugsweise sind in den erfindungsgemäßen Organo(poly)siloxanen die zusätzlich zu den Einheiten der Formel

$$R_3SiO_{1/2}$$

vorliegenden Einheiten zu 100 Molprozent solche der Formel

$$H_2C{=}CR^1COO(CH_2)_nSiO_{3/2} \quad .$$

Häufig sind aber kleinere Mengen anderer Einheiten, wie $RSiO_{3/2}$-, $R_2SiO$- und/oder $SiO_{4/2}$- Einheiten, wobei R jeweils die oben dafür angegebene Bedeutung hat, ohne unvertretbar großen Aufwand nur schwer zu vermeiden.

Die Herstellung der erfindungsgemäßen Organo(poly)siloxanen kann in an sich für die Herstellung von mischpolymeren Organopolysiloxan bekannter Weise durch Hydrolyse von Silan der Formel

$$H_2C{=}CR^1COO(CH_2)_nSiX_3 \quad ,$$

wobei $R^1$ und n jeweils die oben dafür angegebene Bedeutung haben und X Chlor oder Brom oder einen SiOC-gebundenen organischen Rest, für den oben bereits Beispiele angegeben wurden, bedeutet, in Gegenwart von Disiloxan der Formel

$$R_3SiOSiR_3 \quad ,$$

wobei R die oben dafür angegebene Bedeutung hat, in Gegenwart von anorganischer Säure, wie Salzsäure, erfolgen. Die erfindungsgemäßen Organo(poly)siloxane können aber auch beispielsweise in an sich bekannter Weise durch Mischhydrolyse von Silan der Formel

$$H_2C{=}CR^1COO(CH_2)_nSiX_3 \quad ,$$

wobei $R^1$, X und n jeweils die oben dafür angegebene Bedeutung haben, und Silan der Formel

$$R_3SiX \quad ,$$

wobei R und X jeweils die oben dafür angegebene Bedeutung haben, hergestellt werden. Es sei in diesem Zusammenhang betont, daß bei der Herstellung der erfindungsgemäßen Organo(poly)siloxane im Gegensatz zur Herstellung der eingangs erwähnten oligomeren methacrylsubstituierten Alkylsiloxane Silan der Formel

$$H_2C{=}CR^1COO(CH_2)_nSiX_3$$

in handelsüblicher Form mit technischer Reinheit ohne die Vorbehandlung gemäß GB 20 23 628 A eingesetzt werden kann.

Gegenstand der Erfindung sind weiterhin durch Bestrahlung mit Licht vernetzbare Massen, die Organo(poly)siloxan mit Monoorganosiloxaneinheiten der Formel

$$H_2C{=}CR^1COO(CH_2)_nSiO_{3/2} \quad ,$$

worin $R^1$ und n jeweils die oben dafür angegebene Bedeutung haben, Füllstoff, Photosensibilisator und gegebenenfalls weitere Stoffe enthalten, dadurch gekennzeichnet, daß im Organo(poly)siloxan mit Monoorganosiloxaneinheiten der vorstehend angegebenen Formel 1 bis 60 Molprozent Einheiten der Formel

$$R_3SiO_{1/2} \quad ,$$

worin R die oben dafür angegebene Bedeutung hat, vorliegen,

wobei die restlichen Einheiten im Organo(poly)siloxan mit Monoorganosiloxaneinheiten der oben angegebenen Formel mindestens zu 95 Molprozent Monoorganosiloxaneinheiten der oben angegebenen Formel sind.

Vorzugsweise enthalten die erfindungsgemäßen Massen Organo(poly)siloxan mit Monoorganosiloxaneinheiten der Formel

$$H_2C{=}CR^1COO(CH_2)_nSiO_{3/2}$$

in Mengen von 10 bis 30 Gewichtsprozent, bezogen auf das Gesamtgewicht der jeweiligen Masse.

Beispiele für Füllstoffe in den erfindungsgemäßen Massen sind Siliciumdioxyd mit einer Oberfläche von mindestens 50 $m^2/g$, Quarzmehl, Pulver aus Barium enthaltendem Glas, Aluminiumoxyd und Pulver aus Glas, das Lanthan-, Hafnium-, Strontium- oder Tantalverbindungen enthält. Alle diese Füllstoffe können gegebenenfalls auf ihrer Oberfläche Organosilylgruppen, wie Methacryloxypropylsiloxygruppen, aufweisen.

Vorzugsweise enthalten die erfindungsgemäßen Massen 50 bis 90 Gewichtsprozent Füllstoff, bezogen auf das Gesamtgewicht der jeweiligen Masse.

Beispiele für Photosensibilisatoren sind Benzophenon und substituierte Benzophenone, Benzoin und substituierte Benzoine, Benzil und substituierte Benzile, Acetophenon und substituierte Acetophenone. Einzelne Beispiele für derartige substituierte Verbindungen und für weitere Photosensibilisatoren sind Hydroxydimethylacetophenon (=2-Hydroxy -2-methyl-1-phenylpropan-1-on), 2,4-Bis-(trimethylsiloxy)-benzophenon, 2-Ethoxy-2-methylacetophenon, Trichlorbutylacetophenon, 2-Ethoxy-2-phenylacetophenon, Mesityloxyd, Propiophenon, Xanthon, Thioxanthon, Fluorenon, Benzaldehyd, Fluoren,

Anthrachinon, Carbazol, 3-Methylacetophenon, 4-Methylacetophenon, 4,4'-Dimethoxybenzophenon, 4'-Chlor-4'-benzylbenzophenon, 3-Chlorxanthon, 3,9-Dichlorxanthon, 3-Chlor-8-nonylxanthon, Chloranthrachinon, Michlers Keton, Zimtsäure, Benzoinmethylether, Anthrachinon-1,5-disulfonsäuredinatriumsalz, 2-Naphthalinsulfonylchlorid, Benzilketale und Hydroxybenzophenone.

Vorzugsweise enthalten die erfindungsgemäßen Massen Photosensibilisator in Mengen von 0,5 bis 5 Gewichtsprozent, insbesondere 1 bis 2 Gewichtsprozent, jeweils bezogen auf das Gesamtgewicht der jeweiligen Masse.

Beispiele für weitere Stoffe, welche die erfindungsgemäßen Massen enthalten können, sind als Beschleuniger der Vernetzung durch Licht wirkende Amine, wie Dimethylethanolamine, Triethylanolamin und 2-(Dimethylamino)-ethylbenzoat.

Besonders wichtige Beispiele für weitere Stoffe, welche die erfindungsgemäßen Massen enthalten können, sind Acrylsäure und Methacrylsäure, rein-organische Ester dieser Säuren, wobei Triethylenglykoldimethacrylat besonders bevorzugt ist, und andere Derivate dieser Säuren. Weitere Beispiele für Ester der Acryl- und Methacrylsäure und andere Derivate dieser Säuren, welche in den erfindungsgemäßen Massen als weitere Stoffe vorliegen können, sind Ethylacrylat, Methylacrylat, Propylacrylate, Butylacrylate, Methylmethacrylat, Hydroxymethylmethacrylat, Hydroxypropylmethacrylat, 2-Ethylhexylmethacrylat, Cyclohexylacrylat, Acrylamid, Methacrylamid, Acrylnitril, Methacrylnitril, Ethylenglykoldiacrylat, Ethylenglykoldiacrylat, Ethylenglykoldimethacrylat, Diethylenglykoldiacrylat, Diethylenglykoldimethacrylat, Tetraethylenglykoldiacrylat, Tetraethylenglykoldimethacrylat und 2,2'-Propanbis-[3-(4-phenoxy)-1,2-hydroxypropan-1-methacrylat].

Vorzugsweise enthalten die erfindungsgemäßen Massen Acrylsäure, Methacrylsäure, rein-organische Ester dieser Säuren

oder andere Derivate dieser Säuren in Mengen von 1 bis 50 Gewichtsteilen je Gewichtsteil Organo(poly)siloxan mit Monoorganosiloxaneinheiten der Formel

$$H_2C=CR^1COO(CH_2)_nSiO_{3/2} \quad .$$

Die erfindungsgemäßen Massen vernetzen bei Bestrahlung mit Licht mit einer Wellenlänge von höchstens 500 nm (Nanometer).

Die erfindungsgemäßen Massen eignen sich zur Füllung von Hohlräumen in menschlichen und tierischen Zähnen, wobei bei Sichtplomben von besonders großem Vorteil ist, daß die erfindungsgemäßen Massen bei der Vernetzung Produkte ergeben, welche sich unter weiterem Lichteinfluß nicht verfärben. Die erfindungsgemäßen Massen eignen sich weiterhin als Knochenzement und zur Verklebung von Körpern aus silikatischen Werkstoffen, wie Glas oder Porzellan.

In den folgenden Beispielen beziehen sich alle Angaben von Prozentsätzen auf das Gewicht, soweit nichts anderes angegeben ist.

Beispiel 1

Zu einer Lösung von 1240 g (5 Mol) gamma-Methylacryloxypropyltrimethoxysilan und 108 g (0,67 Mol) Hexamethyldisiloxan in 1250 g Toluol werden 146 g 1-n-Salzsäure (0,15 Mol HCl) und 130 g(7,2 Mol) entionisiertes Wasser gegeben. Die so erhaltene Mischung wird unter Rühren eine Stunde zum Sieden unter Rückfluß erwärmt und dann mit 15 g (0,15 Mol) Calciumcarbonat neutralisiert. Aus der so erhaltenen Mischung werden nach dem Filtrieren die bei 130 Pa (abs.) bis zu 80°C siedenden Bestandteile abdestilliert.

Es werden 910 g, d.h. 90 % der Theorie, eines farblosen, klaren Öls mit einer Viskosität von 1100 mPa.s bei 25°C erhalten, während das Oligomere von Beispiel 1 der GB 20 23 628 A eine Viskosität von 11 000 mPa.s bei 25°C hat. Gemäß $^1$H-NMR-Spektrum beträgt das Verhältnis von Methacryloxypropylsiloxaneinheiten zu Trimethylsiloxaneinheiten 3,8 : 1. Der Gehalt an Si-gebundenen Hydroxylgruppen beträgt 3,2 %. Das Öl bleibt auch nach 6 Monaten Lagerung bei Raumtemperatur klar und ändert seine Viskosität nicht, während das Oligomere von Beispiel 1 der GB 20 23 628 A unter den gleichen Lagerbedingungen trüb wird und geliert.

Beispiel 2

a) eine Masse aus
    24,7 % des gemäß Beispiel 1 hergestellten Öls
    4,9 % Triethylenglykoldimethacrylat
    3,6 % pyrogen erzeugtem Siliciumdioxyd mit einer Oberfläche von 200 $m^2/g$
    65,8 % Quarzmehl
    1 % Hydroxydimethylacetophenon

härtet bei Belichtung mit einer handelsüblichen Ultraviolett-Lampe ("Litema Pluraflex UV 200",wobei es sich bei "Litema Pluraflex" um ein Registriertes Warenzeichen handeln dürfte) innerhalb von 30 Sekunden bis in eine Tiefe von 2,1 mm aus.

b) Die vorstehend unter a) beschriebene Arbeitsweise wird wiederholt mit der Abänderung, daß die Masse zusätzlich 0,6 %, bezogen auf das Gesamtgewicht der übrigen Bestandteile, Triethanolamin enthält.

Die Masse härtet bei Belichtung mit der handelsüblichen Ultraviolettlampe innerhalb von 30 Sekunden bis in eine Tiefe von 2,9 mm aus.

Die gemäß a) und b) bereiteten Massen sind unter Ausschluß von Licht mindestens 6 Monate bei Raumtemperatur beständig.

Gemessen an Probekörpern gemäß Specification No. 27 for direct filling resins der New American Dental Association haben die gemäß a) und b) vernetzten Produkte folgende Eigenschaften:

| | |
|---|---|
| Druckfestigkeit: | 249 N/mm² |
| Wasseraufnahme: | weniger als 1 mg/cm² |
| Ausdehnungskoeefizient: | 44 x $10^{-6}$/°C |

Bei 4 Monate währender Belichtung Belichtung tritt keine Verfärbung der gemäß a) und b) vernetzten Produkte auf.

Vergleichsversuch

Die in Beispiel 2 unter a) beschriebene Arbeitsweise wird wiederholt mit der Abänderung, daß 24,7 % des Oligomeren von Beispiel 1 der GB 20 23 628 A anstelle der 24,7 % des gemäß diesem Beispiel 1 hergestellten Öls verwendet werden.

Gemessen an Probekörpern gemäß Specification No. 27 der New American Dental Association hat das Produkt folgende Eigenschaften:

| | |
|---|---|
| Druckfestigkeit: | 123 N/mm² |
| Wasseraufnahme: | 1,2 mg/cm² |
| Ausdehnungskoeffizient: | 57 x $10^{-6}$/°C |

0156036

## Patentansprüche:

1. Organo(poly)siloxane mit Monoorganosiloxaneinheiten der Formel

$$H_2C{=}CR^1COO(CH_2)_nSiO_{3/2} \quad ,$$

worin $R^1$ Wasserstoff oder einen Alkylrest mit 1 bis 4 Kohlenstoffatomen und n eine ganze Zahl im Wert von 2,3 oder 4 bedeutet,
dadurch gekennzeichnet,
daß sie 1 bis 60 Molprozent Einheiten der Formel

$$R_3SiO_{1/2} \quad ,$$

worin R gleiche oder verschiedene SiC-gebundene organische Reste bedeutet, enthalten, wobei die restlichen Einheiten mindestens zu 95 Molprozent Monoorganosiloxaneinheiten der oben angegebenen Formel sind.

2. Organo(poly)siloxaneinheiten nach Anspruch 1,
dadurch gekennzeichnet,
daß sie 15 bis 25 Molprozent Einheiten der Formel

$$R_3SiO_{1/2}$$

und 2 bis 5 Gewichtsprozent Si-gebundene Hydroxylgruppen enthalten.

3. Durch Bestrahlung mit Licht vernetzbare Massen, die Organo(poly)siloxaneinheiten mit Monoorganosiloxaneinheiten der Formel

$$H_2C{=}CR^1COO(CH_2)_nSiO_{3/2} \quad ,$$

worin $R^1$ und n jeweils die oben dafür angegebene Bedeutung haben, Füllstoff, Photosensibilisator und gegebenen-

falls weitere Stoffe enthalten,
d a d u r c h g e k e n n z e i c h n e t,
daß sie Organo(poly)siloxan nach Anspruch 1 enthalten.